(19) Europäisches Patentamt
European Patent Office
Office européen des brevets

(11) Publication number: **0 120 590**
**B1**

(12) **EUROPEAN PATENT SPECIFICATION**

(45) Date of publication of patent specification: **29.07.87**

(51) Int. Cl.$^4$: **C 07 C 1/04**

(21) Application number: **84301065.3**

(22) Date of filing: **20.02.84**

# (54) Production of pipeline gas from sulfur containing raw or synthesis gas.

(30) Priority: **03.03.83 US 471640**

(43) Date of publication of application:
**03.10.84 Bulletin 84/40**

(45) Publication of the grant of the patent:
**29.07.87 Bulletin 87/31**

(84) Designated Contracting States:
**AT BE DE FR GB IT NL**

(56) References cited:
**DE-A-1 545 470**
**DE-A-2 747 517**
**DE-A-2 907 943**
**US-A-3 904 386**
**US-A-3 928 000**

(73) Proprietor: **GAS RESEARCH INSTITUTE**
**8600 West Bryn Mawr Avenue**
**Chicago Illinois 60631 (US)**

(72) Inventor: **Happel, John**
**69 Tompkins Avenue**
**Hastings-on-Hudson New York, 10706 (US)**
Inventor: **Hnatow, Miguel A.**
**45 Cedar Road**
**Caldwell New Jersey, 07006 (US)**
Inventor: **Bajars, Laimonis**
**3 Harrisons Lane**
**Princeton Junction New Jersey, 08550 (US)**

(74) Representative: **Logan, Allan Beattie**
**33, Audley Road**
**Ealing London W5 3ES (GB)**

**EP 0 120 590 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Field of invention

This invention relates to the conversion of coal and carbonaceous materials to natural gas substitutes, and particularly to processes in which raw gas or synthesis gas produced by primary gasification is converted into methane-rich, pipeline-quality gas.

Description of prior art

A number of processes are known for the production of methane-rich gas by the gasification and subsequent methanation of coal or other carbonaceous materials.

Some examples of gasification processes for coal are known as the dry bottom Lurgi, slagging Lurgi and HYGAS processes. The $H_2/CO$ ratio in the synthesis gas produced by these processes varies from about 0.4 to 2.2. The Lurgi process has been selected by a number of firms for construction of the first commercial gasification units in the United States because it has been operated for a long time on a commercial scale. It is based on the steam-oxygen gasification under pressure of sized coal particles in a mechanically stirred fuel bed. The effluent gases from the gasifier are quenched to remove tars and oils and the resulting gases are then passed through a carbon monoxide shift converter where part of the carbon monoxide reacts with steam to produce hydrogen and carbon dioxide by the so-called water gas shift reaction. The next stage consists of the removal of acid gases and residual sulfur compounds followed by passage through iron oxide boxes. The slagging Lurgi process is similar to the commercial Lurgi process described above except that the gasifier is modified to accept caking coals and operates at a higher temperature. Coal is crushed, screened to remove fines and then fed to the gasifier through coal locks. The bottom half of the gasifier is different from the conventional Lurgi gasifier. According to a British Gas Corporation development, steam and oxygen are injected through tuyeres into the high temperature reaction zone. Ash is removed in the form of liquid slag through a tap and dropped into a quench vessel where it is removed as a glasslike frit. Operating conditions of the gasifier are 400—600 psi (27 to 41 atmospheres) and about 2300°F (1260°C). Shift conversion and purification steps similar to those of the conventional Lurgi process are employed in further processing. The HYGAS process of the Illinois Institute of Gas Technology in Chicago is based on the hydrogenation of a fluidized bed of specially prepared coal. The latter is introduced as a slurry into the top layer of three superposed fluidized beds. Hot hydrogen enters the bottom bed and fluidizes all three beds, evaporating at the same time the oil from the slurry that was injected into the top. The fluid bed gasifier is maintained at a pressure of 75—90 atm and this is conducive to the formation of methane during the gasification process. The ungasified portion of the coal is obtained in the form of a reasonably reactive char which is withdrawn from the bottom of the bed and transferred to a hydrogen generator. The latter is normally operated by injecting a steam/oxygen mixture into the fluidized char, but can in the absence of oxygen operate on steam alone, provided the bed is electrically heated. A third possible method of hydrogen production, based on the steam-iron reaction and a subsequent reduction of the iron by means of char, has also been proposed. This process also involves the employment of a raw gas purifier.

Usually one finds that in the above and similar gasification processes, apart from methane, two low calorific value gases are produced simultaneously. Oxygenation and steam reforming both produce carbon monoxide, the calorific value of which is about 300 Btu/cu ft. (42020 kg-calories/cu. m.), and hydrogen, which also has a heating value of around 300 Btu/cu ft. (42020 kg-calories/cu. m.). In order to convert these low calorific gases into methane, the carbon monoxide can be reacted with hydrogen. In the usual methanation process using nickel catalyst, a $H_2/CO$ ratio of about 3 is used. This means that either extra hydrogen must be introduced or that there will remain an excess of surplus carbon monoxide. Carbon dioxide can also react with hydrogen to produce methane, but since production of hydrogen usually involves the conversion of carbon monoxide to carbon dioxide by the shift reaction, this reaction is not usually advantageous. Carbon dioxide typically is removed by absorption in alkaline absorbents and this constitutes one of the major costs in obtaining high heating value gases. In all processes described above, if the aim is to produce synthetic natural gas, the carbon monoxide is hydrogenated.

Methanation, as an industrial process, has been known for many years since it is an essential gas purification step in the synthesis of ammonia from hydrogen and nitrogen. Nickel has generally been the active component in the production of synthetic natural gas (SNG) from synthesis gas produced by primary gasification processes such as those described above.

Although nickel is a very active catalyst it possesses a number of disadvantages. It is deactivated irreversibly by the presence of very small proportions of sulfur compounds in the feed gas. Another problem in the employment of nickel methanation catalysts is the loss in activity due to carbon deposition. Carbon formation is favored at the high methane concentrations required for production of methane, in part due to the reaction:

$$CH_4 \rightleftharpoons 2H_2 + C \qquad (1)$$

This reaction, if it occurs, again irreversibly poisons the nickel catalyst. Generally it is necessary to employ feed gas with high ratios of $H_2/CO$, equal to at least about 3 (on a molar or volumetric basis), to avoid this possibility. Generally, operating conditions involve temperatures below 400°C and pressures up to 1000 psi (68 atmospheres).

Under these conditions the predominant reaction for methane formation over nickel catalysts is:

$$3H_2+CO \rightleftharpoons CH_4+H_2O \quad (2)$$

The hydrogen for the high $H_2CO$ ratio needed for reaction (2) can be supplied by the water shift reaction:

$$H_2O+CO \rightleftharpoons CO_2+H_2 \quad (3)$$

One way to avoid the necessity of using a separate water gas shift reaction step (reaction (3)) is to conduct the methanation in the presence of a large excess of steam (Roberts, F. F. S., Hibden, D., Brooks, C. T., Sudburg, J. D., Paper IGU/Bz-79, 14th World Gas Conference, Toronto, International Gas Union, Paris, 1979). If such large excess of steam is supplied, it is then possible to apparently promote direct formation of methane (by a combination of reactions (2) and (3) in the known prior art):

$$2CO+2H_2 \rightleftharpoons CH_4+CO_2 \quad (4)$$

Reaction (4) does not involve water at all. To avoid water formation with nickel catalysts it is therefore necessary for a large concentration of steam to be maintained so that water formed by reaction (2) is consumed by reaction (3). This introduction of steam also serves to suppress carbon formation with these catalysts.

There has also been considerable effort devoted to the attempt to reduce sulfur sensitivity of nickel catalysts and/or substitute other catalysts which would be less affected by sulfur. One proposal is discussed in U.S. Patent 3,928,000 (Child, et al.). The proposal involves gasifying a high-sulfur carbonaceous fuel by partial oxidation to obtain a process gas stream which is cooled, cleaned and subjected to catalytic methanation. In the methanation step synthesis gases are to be reacted without any preliminary water gas shift, though water is to be employed in the primary partial oxidation step, thereby affording some increase in the $H_2/CO$ ratio. The methanation catalyst is a typical Co/Mo/Al hydrodesulfurization catalyst, available commercially. The feed to the methanation reactor (Example I of the Child et al. patent) is said to have a $H_2/CO$ ratio, equal to 1.76, which is still substantially higher than would correspond to the $H_2/CO=1/1$ ratio for the direct methanation reaction, Equation (4). Operation at 98 atmospheres (1440 psi) is said to result in a product stream which is scrubbed to remove the gases $CO_2$, $H_2S$, COS and $H_2O$. This product is said to contain 3.8 vol.% of CO. It is next to be processed (Example II of the Child et al. patent) by addition of 11.4 vol.% of steam and passing over a fixed bed conventional water-gas shift catalyst consisting of a mixture of copper and zinc oxides. This is said to reduce the CO content to 1.88 vol.% with simultaneous production of hydrogen, so that the hydrogen content of the exit gases from the shift converter was 5.63 vol.%, corresponding to a $H_2/CO$ ratio of 3/1. After removal of $H_2O$ and $CO_2$ the process gas was to be introduced into a conventional adiabatic catalytic methanation zone using a nickel containing catalyst. $CO_2$ was said to be again removed from the product through this methanation reaction to obtain the final product SNG.

Thus, the processing sequence proposed by Child et al. uses three acid gas removal steps. Acid gas removal is one of the most expensive operations, costing far more than the catalytic processing steps themselves. Also, the use of additional steam both in the original gasification and the subsequent water gas shift adds considerable cost to the process.

Another attempt to produce pipeline quality gas economically by so-called combined shift and methanation had been proposed earlier, in U.S. Patent No. 3,904,386 (Grabowski, et al.). In this case the synthesis gas feed is said to be derived from a coal gasifier. The cooled synthesis gas is to be introduced into a combined shift and methanation system where the mixture comes in contact with a catalyst at a temperature between 500°F (260°C) and 1050°F (565°C) and a pressure between 500 psig (34 atmospheres) and 2000 psig (136 atmospheres). The catalyst employed is said to be based on molybdenum and alumina, although addition of cobalt and other promoters are mentioned, so that the catalyst employed seems to be similar to that specified in the Child et al. patent. Finally, as in the Child et al. patent, the sulfur-free gas is to be treated to produce a gas of SNG pipeline quality through conventional fixed bed methanation using a nickel catalyst.

In the Grabowski et al. proposal, a much lower ratio of $H_2/CO$ in feed gas to the combined shift and methanation reaction is to be employed than in the Child et al. patent, namely $H_2/CO=0.66$ (from Table II of U.S. Patent No. 3,904,386). Thus, there is not enough hydrogen present in this case for direct methanation, as in reaction (4). In order to supply sufficient hydrogen, 0.5 vol. of steam must be fed to the combined shift-methanation reactor for each volume of dry gas. In order to avoid deactivating the catalyst by this large addition of steam, it is necessary to recycle a portion of dry product gas from the shift-methanation reactor back to the feed. This feature is believed to be an essential part of the process and, of course, adds substantially to the cost of that process.

In German published Patent Specification DE—A—1 545470 there is described a process for converting synthesis gas containing hydrogen and carbon monoxide and sulfur containing gas to methane-rich pipeline-quality final product gas, comprising reacting hydrogen and carbon monoxide in the presence of a first catalyst and in the presence of said sulfur containing gas in a direct methanation reaction to produce

intermediate product gas rich in methane and containing residual hydrogen and carbon monoxide as well as acid gases, removing acid gases from the intermediate product gas, and reacting the residual hydrogen and carbon monoxide in the gas in the presence of a second catalyst to increase the methane content of the gas and thereby convert it to a methane-rich pipeline-quality final product gas. No ratio of hydrogen to carbon monoxide in the synthesis gas is given in this specification but it is stated that a carbon monoxide-rich gas, for example water gas and higher hydrocarbon may be added. The catalyst for the first methanation reaction is said to be a molybdenum catalyst for example cobalt molybdenum, nickel molybdenum or nickel-cobalt molybdenum, which is the same type of catalyst as suggested by Child et al.

## Summary of the invention

The invention is in the field of conversion of carbonaceous materials or products thereof into natural gas substitutes, and is useful for a final product gas which is rich in methane and has a sufficiently low content of carbon monoxide and hydrogen sulfide to be useful as pipeline quality gas either directly or when blended with other gases.

This invention makes use of the discovery that oxysulfide catalysts based on the transition elements W, Mo, and V have much higher activity for direct methanation reaction (4) than nickel catalysts while being relatively less active for the water gas shift reaction. Direct methanation is the predominant reaction over these catalysts even in the presence of substantial concentrations of water and carbon dioxide. A process sequence employing the reaction takes advantage of this discovery.

In an exemplary example of a process embodying the invention, the synthesis gas for direct methanation contains hydrogen and carbon monoxide in the ratio of 0.9—1.3, and also sulfur containing gas and up to about 30 vol.% carbon dioxide on water-free basis, and preferably but not necessarily some methane. Hydrogen and carbon monoxide in the syntheses gas react in the presence of a direct methanation catalyst to produce methane and carbon dioxide, to thereby convert the synthesis gas to a methane-rich intermediate product gas which contains residual amounts of hydrogen and carbon monoxide, as well as carbon dioxide and sulfur containing gas. This intermediate product gas is scrubbed to remove acid gases, such as carbon dioxide and hydrogen sulfide, and the scrubbed gas is polished by methanation over a catalyst such as nickel, in which the residual hydrogen and carbon monoxide react to produce methane and water and thereby convert the scrubbed gas into a final product gas which is methane-rich, in that it contains at least 75 vol.% methane, and is suitable for direct pipeline use or for lending into pipeline gas because its carbon monoxide content is no more than about 0.1 vol.% and its sulfur content is no more than about 5 ppm.

If syntheses gas having the desire $H_2/CO$ ratio (about 0.9—1.3) is not available, it can be produced by preconditioning raw gas from processes such as certain types of coal gasification. If the raw gas has too high a ratio of hydrogen to carbon monoxide, e.g. a ratio greater than about 1.3, it can be converted to synthesis gas having the preferred range of about 0.9—1.3 by a reverse water gas shift reaction in the presence of a sulfur-insensitive catalyst, in which hydrogen and carbon dioxide in the raw gas react to produce carbon monoxide and water until the hydrogen to carbon monoxide ratio drops to the preferred range and preferably to about one. Conversely, if the raw gas hydrogen to carbon monoxide ratio is less than the preferred range of about 0.9—1.3, it can be subjected to a water gas shift reaction over a sulfur-insensitive catalyst with the addition of steam, in which carbon monoxide and water react to produce hydrogen and carbon dioxide, to thereby raise the $H_2/CO$ ratio to the preferred range, and preferably to about one.

If the intermediate product gas from the direct methanation reaction has residual hydrogen to carbon monoxide ratio of less than about 3, i.e., has insufficient hydrogen content for efficient polishing methanation over a catalyst such as Ni, its ratio can be raised prior to the acid gas scrubbing step by subjecting it to a post-conditioning step involving a water gas shift reaction over a sulfur-insensitive catalyst, in which residual carbon monoxide reacts with steam to produce hydrogen and carbon dioxide until the $H_2/CO$ ratio increases to at least about 3.

The scrubbed gas is polished by a final methanation reaction, e.g., reaction (2) over a Ni catalyst, to produce the methane-rich final product gas which can be used as a pipeline gas either directly or in a blend.

In the alternative, the above steps following direct methanation can be replaced by cryogenic separation which involves feeding $CO_2$ and $H_2$ separated from the intermediate product gas back to the direct methanation step and supplying separated methane in the form of, e.g., liquid (synthetic) natural gas.

As another alternative, a preliminary direct methanation of the raw gas can be added, for partial methanation prior to any needed preconditioning step preceding the main direct methanation step.

If a gas contains approximately equal proportions of $H_2$ and CO, the occurrence of the direct methanation reaction, Equation (4), is favored. Actually a slight excess of $H_2$ is preferable because the reaction shown by Equation (2) occurs to minor extent. For gases such as those produced by the HYGAS process after the usual quench it is possible and economical to pass the raw gas containing the acid gases $H_2S$ and $CO_2$ directly through a reactor system containing catalysts such as those described in U.S. Patents Nos. 4,151,191 and 4,320,030. The direct methanation reaction converts to methane up to 95% of the CO in the feed gas from the HYGAS quench. It is then only necessary to adjust the $H_2/CO$ ratio of the intermediate product so that a final clean-up (polishing) operation can be conducted to reduce the CO content to the

drastic pipeline gas specification of less than 0.1% vol. A convenient way to accomplish this is to drop the temperatures of the effluent from the methanation step and pass it over a sulfur-insensitive water gas shift catalyst. Several such catalysts are available including the formulation given in our Patent 4,151,191. No or little addition of water is necessary with active catalysts which can bring the reaction close to equilibrium at 300°C. The gas then should have a $H_2/CO$ ratio of greater than 3/1. Following this the $CO_2$ produced both in the last step and the previous methanation is removed along with sulfur containing gases by scrubbing with an alkaline solvent. The effluent gas is methanated in a clean-up operation using a conventional nickel catalyst to produce a high Btu synthetic natural gas. This gas is produced by addition of very minor proportions of process steam and involves only one acid gas removal step.

If the raw gas contains a higher ratio of $H_2/CO$, such as for example that produced by the dry bottom Lurgi gasifier which has a $H_2/CO$ ratio equal to about 2, it can be preconditioned (prior to the direct methanation step) by $CO_2$ methanation. For preconditioning, the raw gas is passed over a sulfur-insensitive water gas shift catalyst at an elevated temperature (600°) at which the water gas shift equilibrium constant is small. The $H_2/CO$ ratio is thus reduced to a lower value and the resulting synthesis gas ($H_2/CO \approx 0.9$—1.3) can be processed as previously described.

An alternative scheme is to immediately use the raw gas in the direct methanation reaction. The conversion of CO is high but excess hydrogen is produced. In the subsequent acid gas removal step, some $CO_2$ is left in the product gases. These gases are passed to a nickel catalyst step in which not only CO but also $CO_2$ methanation ($4H_2+CO_2 \rightleftharpoons CH_4+2H_2O$) is conducted so as to use up the excess hydrogen. In both cases only one acid gas removal step is required.

If the $H_2/CO$ ratio is low (e.g., below about 0.9), such as in the raw gas produced by the slagging Lurgi gasifier, which has a $H_2/CO$ equal to about 0.4, after the usual quench the gas is passed at a low temperature (about 300°C) over a water gas shift catalyst in order to raise the hydrogen/carbon monoxide ratio. It is then subsequently processed as previously described.

## The drawing

The sole Figure is a flowchart of processes embodying aspects of the invention.

## Description of preferred embodiments

The term "raw gas" used in this specification refers to a mixture which includes carbon monoxide, hydrogen and preferably some methane gases. Examples of raw gases are the effluents of the primary gasification steps in processes such as the dry bottom Lurgi, slagging Lurgi, HYGAS, or other similar processes. The raw gas can contain from about 0.4 to 2.2 volumes of hydrogen for volume of carbon monoxide, and up to about 3 vol.% of $H_2S$. It can also contain $CO_2$ and/or water, along with smaller quantities of nitrogen, aromatic hydrocarbons and phenols derived from the carbon source. The term "synthesis gas" is used herein to mean a raw gas in which the $H_2/CO$ ratio is, or has been adjusted to, a figure in the range of 0.3—1.7, and preferably 0.9—1.3. The term "intermediate product gas" refers herein to the product of the direct methanation step.

Referring to the flow diagram, source 10 of raw gas can be a conventional gasifier process such as HYGAS, slagging Lurgi, dry bottom Lurgi, which includes the conventional "quencher" step, at a quenching temperature dictated by the desired quality of the raw gas and $H_2$ to CO ratio of the stream, and a conventional water and oil scrubbing operation to remove tar, oils, phenol ammonia and other coal by-products.

Depending on the $H_2$ to CO ratio of the raw gas, a preconditioning step can be used to adjust this ratio to around 0.9 to 1.3 for more effectively employing direct methanation of CO and $H_2$ to produce $CH_4$ and $CO_2$ without employing steam.

If the $H_2$ to CO ratio in the raw gas stream from step 10 is lower than about 0.9, as in the case of the slagging Lurgi coal gasification process, a gas water shift reaction (3) is used at step 12 to shift some of the CO in the raw gas to $H_2$ to bring the ratio up to within about 0.9—1.3, and preferably to around 1.0. If the $H_2$ to CO ratio is greater than about 1.3, as in the case of dry bottom Lurgi coal gasification process, at step 14 $CO_2$ in the raw gas is reacted with hydrogen to form CO and water by the reverse of the water gas shift reaction (3) to bring the ratio down to within about 0.9—1.3, and preferably to about 1. These reactions occur readily over commercially available water gas shift catalysts that are insensitive to sulfur compounds. A commercially available unit for either of these preconditioning steps 12 and 14 can be operated at a range of space velocities from 1,000 to 15,000 V/V/HR(STP) and compatable temperatures and pressures in a manner known in the art. The output of the preconditioning step (12 or 14) is "synthesis gas", as the term is used in this specification. Of course, if the raw gas has an $H_2/CO$ ratio in the range specified for synthesis gas, no preconditioning is needed, although some scrubbing to remove tars, oils, ammonia liquid and other by-products can still be carried out if desired.

The synthesis gas enters at step 16 a reactor system that promotes a direct methanation reaction in which the bulk of the methanation occurs. The direct methanator reactor system can take various forms. It can use a series of plug flow reactors arranged and engineered in a configuration for effective heat removal, or a combination of fluidized and fixed bed reactors. The direct methanation process can be conducted at temperatures in the range of about 450°F (232°C) to 1200°F (650°C) and at pressures in the range of 150 to 2,000 psig (10 to 136 atmospheres) and at space velocities in the range of 1,000 to 25,000

V/V/HR (STP). Since the final product gas is typically delivered to the pipeline at 1,000 psig (68 atmospheres), it can be advantageous to operate the direct methanation reactor at step 16 at high pressures in order to deliver the final product gas at pipeline specification without additional compression. The specific operation of the reactor depends on the activity of the catalysts employed.

The synthesis gas fed to the direct methanation system at step 16 can contain up to about 30 vol.% of $CO_2$ on a water free basis, depending on the basic gasification (and any preconditioning) system which supplies it. For conversion of the synthesis gas to methane, the gas entering the direct methanation reactor at step 16 does not need a $H_2$ to CO ratio greater than about 1, which is the requirement for direct methanation. Higher ratios of $H_2$/CO in the feed to step 16 typically result in an increase in the $H_2$ to CO ratio in the product of that step.

The catalyst employed in the direct methanation system at step 16 is preferably a sulfided catalyst, based on one or more elements of the group consisting of tungsten, molybdenum and vanadium and being supported and/or promoted by one or more selected from the group Al, Si, B, Ce, Ti, Zr. A particularly effective catalyst is described in our co-pending patent application S.N. 431,192 (U.S. Patent 4,491,639), entitled High Activity Transition Metal Catalysts and Methods for Making Them, and filed on September 30, 1982.

The intermediate product gases leaving the direct methanator contain some small amount of $H_2$ and CO, the ratio depending on the ratio of the $H_2$ and CO in the feed to step 16. A postconditioning reactor can be used, if needed, to adjust the $H_2$ to CO ratio to a level suitable for a polishing methanation reaction at step 22. The reaction at step 22 can be, for example, over a nickel catalyst, which is most effective at ratios of $H_2$ to CO somewhat in excess of 3 to 1. The specific ratio depends on the desired heating value of the product gas. If the highest heating value is to be obtained, a ratio slightly greater than 3 is desired.

If postconditioning is needed or desired, to bring up the $H_2$/CO ratio, a postconditioning reactor is used at step 18 in which some of the CO in the intermediate product gas is shifted, by a conventional water gas shift reaction preferably over a sulfur-insensitive catalyst, to produce additional hydrogen, and preferably at low temperatures. If needed for this reaction, water can be added at this point. Such $H_2O$ addition is a function of the degree of conversion that is necessary and the activity of the catalyst employed.

Whether a postconditioning reactor is employed or not, the gases next are passed to a solvent treater system used at step 20 to remove acid gases. Step 20 involves the use of conventional processes for sulfur compounds such as $H_2S$ and $CO_2$ removal. Such known processes can involve refrigeration and physical or chemical absorption with solvents such as methyl alcohol, n-methyl pyrolidone, monoethanol amine, diethanolamine, triethanol amine, or hot potassium carbonate. After absorption the solvent is regenerated by stripping the acid gases.

In addition to selective solvent step for the removal of sulfur compounds such as $H_2S$ and $CO_2$, it can be advantageous to pass the gases through zinc oxide boxes for final removal of $H_2S$ down to approximately 0.2 ppm (parts per million by volume) using known processes. Indeed, this can be desirable to avoid catalyst poisoning should a nickel catalyst be employed in the final polishing step 22. However, if sulfur tolerant catalysts are used in the final polishing step 22, such zinc oxide guard is not needed.

Following conventional scrubbing of acid gases at step 20, the scrubbed intermediate product gas stream entering the final polishing step 22 typically contains from about 75 vol.% of methane up to about 96 vol.% of methane. It can contain up to about 5 vol.% of CO and up to 20 vol.% $H_2$. The specific ratios and amounts depend on the $H_2$/CO ratio of the raw gas and the degree of any pre- and postconditioning that may be required. The composition of the gas entering the polishing reactor at step 22 should be such as to ensure that a single pass adiabatic operation is feasible without excessive temperature rise. The $CO_2$ content will normally be less than 1 vol.%.

Following the polishing conversion at step 22 (e.g., by a known methanation reaction over Ni catalyst) the final product gas is now of pipeline quality. It typically contains about 90 vol.% $CH_4$, less than 10 vol.% $H_2$, small amounts of $CO_2$ and other gases which can be introduced in the original raw or synthesis gas (such as $N_2$), and small proportions of other hydrocarbons present in the synthesis gas and also produced during methanation. The sulfur content is typically less than about 0.1 ppm, and the CO content is typically less than about 0.1 vol.%.

As an alternative to steps 18, 20 and 22, the intermediate product gas produced in the direct methanation step 16 can be subjected to crogenic methane separation at step 24. The separated products of the cryogenic methane separation step 24 are carbon monoxide and hydrogen (which can be recirculated back into the direct methanation step 16), pipeline-quality final product gas in the form of liquid synthetic natural gas, and acid gases. The advantages of using cryogenic methane separation in the invented process include avoidance of the need for use of zinc oxide boxes for hydrogen sulfide reduction below 1 ppm, as can be required in connection with nickel methanation as in step 22, elimination of the need for methanation as in step 22 and the fact that the methane from step 24 can be used directly for LSNG and in any event it need not be heated again for polishing methanation.

As another alternative, a preliminary direct methanation, similar to that in step 16, can be carried out at step 26, prior to the preconditioning step 14, to effect partial methanation of the raw gas from a process such as dry bottom Lurgi gasification.

Examples

The following Examples should assist for a better understanding of the invention.

Example 1

This example demonstrates the application of the direct methanation chemistry to process a raw gas that is produced from a HYGAS-type gasifier. See Table 1.

A simulated HYGAS raw gas similar to that reported in a Final Report for the Department of Energy #FE-1772-11 prepared by Dravo Corp. for the Institute of Gas Technology gasifying a Bituminous Pittsburgh #8 coal was used in the laboratory to demonstrate the production of pipeline-quality final product gas. The quenched raw gas (1), which is a synthesis gas in this example because its $H_2/CO$ ratio is within 0.9—1.3, enters the direct methanation process of step 16, where about 92% CO conversion and 57.6% CO selectivity to $CH_4$ is achieved. The effluent (2) from the direct methanation step 16 enters at step 18 a conventional postconditioning shift reactor carrying out step 18, where about 43% of the CO is shifted with additional water to form $H_2$. The acid gases from the product (4) of the postconditioning step 18 are then removed at step 20 in a conventional scrubbing operation to the desired specifications to produce feed (5) to be further methanated in a conventional reaction over a nickel catalyst at step 22. The product of step 22 after being dried is rich in $CH_4$, has a high caloric value, and meets the specifications of pipeline-quality gas.

TABLE 1
Direct methanation of a HYGAS synthesis gas

| Stream No. | (1) | (2) | (3) | (4) | (5) | (6) |
|---|---|---|---|---|---|---|
| Stream name<br>Component | Quenched raw gas to step 16 | Direct methanation product from step 16 | Feed to post conditioning step 18 | Prod. from post conditioning (feed to step 20) | Feed to polish step 22 | Pipeline* gas (Product from step 22) |
| $H_2$ lb. moles/hr | 12,616 | 1,434 | 1,434 | 1,725 | 1,725 | 614 |
| CO | 8,827 | 675 | 675 | 384 | 384 | 13 |
| $CO_2$ | 9,950 | 13,504 | 13,504 | 13,795 | 138 | 138 |
| $CH_4$ | 7,876 | 12,492 | 12,492 | 12,492 | 12,492 | 12,863 |
| $C_2H_6$ | 276 | 276 | 276 | 276 | 276 | 276 |
| $H_2O$ | — | 1,043 | 1,882 | 1,591 | — | — |
| $COS+H_2S$ | 477 | 477 | 477 | 477 | — | — |

*Caloric value, 987 Btu/sc.ft. (60°F, (16°C), 1 atm. HHV)
1 Btu/sc.ft.=140 kg-calories/cu. m. approximately

Example 2

This example demonstrates the application of the direct methanation chemistry to process a raw gas that is produced from a slagging Lurgi gasifier. See Table 2.

A simulated slagging Lurgi raw gas similar to one reported by Conoco in their report to the Department of Energy, FE-2540-10 (Vol. 1) was used to demonstrate the production of pipeline gas. Since the $H_2$ to CO ratio is low, at about 0.4, some preconditioning of this gas (as in step 12) is desired to bring the $H_2$ to CO ratio to about 1.0. This requires about 16 volume% water in the gas to the precondition reactor. Thus, the raw gas is quenched to a temperature to retain sufficient water in the gas as required for shifting the CO to produce $H_2$ in the preconditioning reactor. Having achieved the desired composition of the quenched gas (1), about 26% of the CO is converted to $H_2$ by shifting with water in the preconditioning reactor at step 12. The resulting synthesis gas (2) then enters the direct methanation process at step 16, where about 90% CO conversion and 50% CO selectivity to $CH_4$ is achieved. The effluent (4) from the direct methanation section enters a post-conditioning shift reactor at step 18, where about 63% of CO is shifted with additional water to form $H_2$. The acid gases are then removed at step 20 from the product (6) of step 18 to the desired specification to enable the gas (7) to be further processed at step 22 in a conventional nickel polishing methanation reactor. The final product gas from step 22 after being dried is rich in $CH_4$ and has a high caloric value and is of pipeline quality.

TABLE 2
Direct methanation of a slagging Lurgi synthesis gas

| Stream No. | 1 | 2 | 3 | 4 | 5 | 6 | 7 | 8 |
|---|---|---|---|---|---|---|---|---|
| Stream name / Component | Feed to pre-conditioning reactor | Product from pre-conditioning reactor | Feed to direct methanation | Product from direct methanation | Feed to post conditioning reactor | Product from post conditioning reactor | Feed to polishing reactor | Pipeline* gas |
| CO lb. moles/hr | 56411.7 | 41556.9 | 41556.9 | 4159.1 | 4159.1 | 1289.3 | 1289.3 | 25.8 |
| $CO_2$ | 6208.0 | 21062.7 | 21062.7 | 39761.5 | 39761.5 | 42831.3 | 213.2 | 213.2 |
| $H_2$ | 29764.5 | 39619.3 | 39619.3 | 2221.4 | 2221.4 | 5091.2 | 5091.2 | 1300.7 |
| $CH_4$ | 5870.6 | 5870.6 | 5870.6 | 24569.4 | 24569.4 | 24569.4 | 24569.4 | 25832.8 |
| $C_2+$ | 482.0 | 482.0 | 482.0 | 482.0 | 482.0 | 482.0 | 482.0 | 482.0 |
| $H_2S+COS$ | 1976.1 | 1976.1 | 1976.1 | 1976.1 | 1976.1 | 1976.1 | 684.4 | 684.4 |
| $N_2$ | 684.4 | 684.4 | 684.4 | 684.4 | 684.4 | 684.4 | | |
| $H_2O$ | 15406.5 | 552.0 | 552.0 | 552.0 | 7494.6 | 4624.9 | | |

*Caloric value about 962 Btu/scf (HHV, 60°F, 1 atm)

Example 3

This example demonstrates the application of the direct methanation chemistry to process a raw gas that is produced from a dry bottom Lurgi gasifier option #1 ($CO_2$ Methanation). See Table 3.

A simulated dry bottom Lurgi raw gas was used to demonstrate the production of pipeline gas. The raw gas composition was taken from the El Paso study conducted by Stearns-Roger Co. in which a New Mexico sub-bituminous coal was gasified in a dry bottom Lurgi gasifier. The raw synthesis gas is first quenched to remove the oils, tars and most of the water and produce quenched raw gas (1). The gas then enters, as feed (2), the direct methanation processing unit at step 16, where about 87% CO conversion and around 50% CO selectivity to $CH_4$ is achieved and gas (3) is produced. The acid gas removal system removes at step 20 from gas (3) substantially all of the sulfur compounds, but only about 90% of the $CO_2$, to produce feed (4) to step 22. This feed (4) enters a conventional nickel type methanation reactor at step 22, where substantially all of the CO and the $CO_2$ are methanated, consuming the majority of the excess $H_2$. The final product gas from step 22 after being dried is rich in $CH_4$ and has a high caloric value.

TABLE 3
Direction methanation of a dry bottom Lurgi synthesis gas option #1 ($CO_2$-methanation)

| Stream # | 1 | 2 | 3 | 4 | 5 |
|---|---|---|---|---|---|
| Stream name / Component | Quenched raw gas | Feed to direct methanation | Product from direct methanation | Feed to 2nd methanator | Pipeline* gas |
| $H_2$ lb. moles/hr | 38860 | 38860 | 22523 | 22523 | 1972 |
| CO | 18675 | 18675 | 2338 | 2338 | 29 |
| $CH_4$ | 10814 | 10814 | 18983 | 18983 | 24698 |
| $CO_2$ | 25939 | 25939 | 34107 | 3416 | 34 |
| $C_2$+ | 943 | 943 | 943 | 943 | 943 |
| $H_2S$+COS | 375 | 375 | 375 | | |
| $H_2O$ | 568 | 568 | 568 | | |

*Caloric value 989.7 Btu/scf (60°F, 1 atm., HHV)

Example 4

This example demonstrates the application of the direct methanation chemistry to process a raw gas that is produced from a dry bottom Lurgi gasifier option #2 (feed preconditioning). See Table 4.

A simulated dry bottom Lurgi raw gas was used. The raw gas composition was taken from the El Paso study conducted by Stearns-Roger Co. in which a New Mexico sub-bituminous coal was gasified in a dry bottom Lurgi gasifier. The raw gas is first quenched to remove the oil and tars and most of the water and produce quenched raw gas (1) which becomes feed (2) to a preliminary direct methanation step 24 which operates similarly to step 16 to effect partial methanation of the raw gas. The product (3) from step 24 is feed (4) to preconditioning step 14, whose product (5) is feed (6) to direct methanation step 16, which effects a high degree of conversion. The product (7) of step 16 already has a high enough $H_2$/CO ratio, so there is no need for post-conditioning at step 18. Instead, product (7) from the direct methanation step 16 is directly supplied to scrubbing step 20, to remove acid gases to a degree allowing effective conventional Ni polishing methanation, and the product of step 20 is feed (8) to polishing step 20, whose product (9) is methane-rich, pipeline-quality gas of high caloric value.

TABLE 4
Direct methanation of a dry bottom Lurgi synthesis gas option #2 (feed pre-conditioning)

| Stream # | 1 | 2 | 3 | 4 | 5 | 6 | 7 | 8 | 9 |
|---|---|---|---|---|---|---|---|---|---|
| Stream name / Component | Quenched raw gas | Feed to 1st Direct methanation unit | Product from 1st direct methanation unit | Feed to preconditioning reactor | Product from conditioning reactor | Feed to 2nd direct methanation unit | Product from 2nd direct methanation unit | Feed to polishing reactor | Pipeline* gas |
| $H_2$ lb. moles/hr | 39047 | 39047 | 29872 | 29872 | 21005 | 26005 | 6235 | 6235 | 551 |
| CO | 18764 | 18764 | 12200 | 12200 | 16067 | 16067 | 1924 | 1924 | 29 |
| $CH_4$ | 10895 | 10895 | 14830 | 14830 | 14830 | 14830 | 23308 | 23308 | 25203 |
| $CO_2$ | 26063 | 26063 | 28693 | 28693 | 24826 | 24826 | 30491 | 309 | 309 |
| $C_2$+ | 947 | 947 | 947 | 947 | 947 | 947 | 947 | 947 | 947 |
| $H_2O$ | 570 | 570 | 1875 | 1875 | 5742 | 5742 | 8556 | | |
| $H_2S$+COS | 377 | 377 | 377 | 377 | 377 | 377 | 377 | | |

*Caloric value 1013.6 Btu/scu (HHV, 60°F, 1 atm.)

**Claims**

1. A process of converting synthesis gas containing hydrogen, carbon monoxide and sulfur containing gas to methane-rich, pipeline-quality final product gas in which the synthesis gas hydrogen and carbon monoxide is reacted in the presence of a first catalyst and in the presence of said sulfur containing gas in a direct methanation reaction to produce intermediate product gas rich in methane and containing residual hydrogen and carbon monoxide as well as acid gases;

scrubbing acid gases from the intermediate product gas; and

reacting residual hydrogen and carbon monoxide in the scrubbed gas in the presence of a second catalyst to increase the methane content of the scrubbed gas and to reduce its carbon monoxide content to no more than about 0.1 vol. % and thereby convert it to methane-rich, pipeline-quality final product gas;

characterized in that the ratio of hydrogen to carbon monoxide in the synthesis gas is in the range between 0.9 to 1.3 and the first catalyst is an oxysulfide catalyst based on the transition elements tungsten, molybdenum and vanadium.

2. A process as in Claim 1, characterized in that said first catalyst is a sulfided catalyst based on one or more of the elements of the group consisting of tungsten, molybdenum and vanadium, and being supported and/or promoted by one or more elements selected from the group Al, Si, B, Ce, Ti, Zr.

3. A process as in Claim 1 or 2, characterized in that raw material is gasified to produce said synthesis gas, having hydrogen and carbon monoxide in said ratio.

4. A process as in Claim 1 or 2, characterized in that raw material is gasified to produce raw gas containing hydrogen and carbon monoxide in a ratio outside the range of 0.9—1.3, and the raw gas is preconditioned to adjust the hydrogen to carbon monoxide ratio therein to produce said synthesis gas.

5. A process as in Claim 4, characterized in that raw material is gasified to produce raw gas containing hydrogen and carbon monoxide in a ratio greater than 1.3, as well as carbon dioxide, and the raw gas is preconditioned by reacting the raw gas hydrogen and carbon dioxide in the presence of sulfur insensitive catalyst, in a reverse water gas shift reaction, to reduce the hydrogen content and increase the carbon monoxide content sufficiently to convert the raw gas to said synthesis gas.

6. A process as in Claim 4, characterized in that the raw gas contains hydrogen and carbon monoxide in a ratio of less than 0.9, as well as water, and the preconditioning step comprises reacting the raw gas carbon monoxide and water in the presence of a sulfur insensitive catalyst, in a water gas shift reaction, to reduce the carbon monoxide content and increase the hydrogen content sufficiently to convert raw gas to said synthesis gas.

7. A process as in Claim 4, characterized in that the raw gas hydrogen and carbon monoxide is reacted in the presence of a sulfur-insensitive catalyst in a preliminary direct methanation reaction without substantial addition of water to effect partial methanation of the raw gas and the resulting gas is fed to said preconditioning step.

8. A process as in any one of Claims 1 to 4, characterized in that said intermediate product gas contains residual hydrogen and carbon monoxide in the ratio of less than 3, as well as water, and said intermediate product gas is postconditioned by reacting the product gas water and carbon monoxide in the presence of a sulfur-insensitive catalyst, in a water gas shift reaction, to raise the hydrogen to carbon monoxide ratio therein to at least 3 prior to feeding the product gas to the acid gas scrubbing step.

9. A process as in Claim 1, characterized in that instead of said last mentioned reaction, the intermediate product gas is subjected to cryogenic methane separation producing separated carbon monoxide and hydrogen fed back to the direct methanation step, separated acid gases and separated methane-rich, pipeline-quality final product gas.

10. A process as in Claim 9, characterized in that said separated final product gas is substantially liquified.

**Patentansprüche**

1. Verfahren zum Unwandeln von Synthesegas, das Wasserstoff, Kohlenmonoxid, und schwefelhaltiges Gas enthält, in ein methanreiches endgültiges Produktgas mit Rohrleitungsqualität, bei dem der Wasserstoff und das Kohlenmonoxid des Synthesegases in Gegenwart eines ersten Katalysators und in Gegenwart des genannten schwefelhaltigen Gases in einer direkten Methanbildungs-Reaktion zur Reaktion gebracht wird, um ein Zwischenproduktgas zu erzeugen, das mit Methan angereichert ist und restlichen Wasserstoff und restliches Kohlenmonoxid sowie saure Gase enthält; bei dem saure Gase aus dem Zwischenproduktgas herausgewaschen werden; und bei dem restlicher Wasserstoff und restliches Kohlenmonoxid in dem gewaschenen Gas in Gegenwart eines zweiten Katalysators zur Reaktion gebracht wird, um den Methangehalt des gewaschenen Gases zu erhöhen und um seinen Kohlenmonoxidgehalt auf nicht mehr als 0,1 Vol.% zu vermindern und es dadurch in methanreiches, endgültiges Produktgas mit Rohrleitungsqualität umzuwandeln, dadurch gekennzeichnet, daß das Verhältnis von Wasserstoff zu Kohlenmonoxid in dem Synthesegas im Bereich zwischen 0,9 bis 1,3 ist, und der erste Katalysator ein Oxisulfid-Katalysator ist, der auf den Übergangselementen Wolfram, Molybdän und Vanadium beruht.

2. Verfahren nach Anspruch 1, dadurch gekennzeichnet, daß der genannte erste Katalysator ein sulfidierter Katalysator ist, der auf einem oder mehreren Elementen aus der aus Wolfram, Molybdän und

Vanadium bestehenden Gruppe beruht, und daß als Träger und/oder Promoter eines oder mehrere Elemente dienen, die aus der Gruppe Al, Si, B, Ce, Ti, Zr ausgewählt sind.

3. Verfahren nach Anspruch 1 oder 2, dadurch gekennzeichnet, daß zur Erzeugung des genannten Synthesegases Rohmaterial vergast wird, das Wasserstoff und Kohlenmonoxid in dem genannten Verhältnis aufweist.

4. Verfahren nach Anspruch 1 oder 2, dadurch gekennzeichnet, daß Rohmaterial vergast wird, um Rohgas zu erzeugen, das Wasserstoff und Kohlenmonoxid in einem Verhältnis außerhalb des Bereiches von 0,9 bis 1,3 enthält, und daß das Rohgas vorbehandelt wird, um das Wasserstoff-Kohlenmonoxid-Verhältnis darin für die Erzeugung des genannten Synthesegases einzustellen.

5. Verfahren nach Anspruch 4, dadurch gekennzeichnet, daß Rohmaterial vergast wird, um Rohgas zu erzeugen, das Wasserstoff und Kohlenmonoxid in einem Verhältnis größer als 1,3 sowie Kohlendioxid enthält, und daß das Rohgas dadurch aufbereitet wird, daß der Wasserstoff und das Kohlendioxid des Rohgases in Gegenwart eines schwefelunempfindlichen Katalysators in einer umgekehrten Wasser-Gas-Verschiebungs-Reaktion zur Reaktion gebracht wird, um den Wasserstoff-Gehalt zu reduzieren und den Kohlenmonoxid-Gehalt ausreichend zu erhöhen, um das Rohgas in das genannte Synthesegas umzuwandeln.

6. Verfahren nach Anspruch 4, dadurch gekennzeichnet, daß das Rohgas Wasserstoff und Kohlenmonoxid in einem Verhältnis von weniger als 0,9 sowie Wasser enthält, und daß der Verbehandlungsschritt die Reaktion des Rohgas-Kohlenmonoxids und des Wassers in Gegenwart eines für Schwefel unempfindlichen Katalysators in einer Wasser-Gas-Verschiebungs-Reaktion umfaßt, um den Kohlenmonoxid-Gehalt zu vermindern und den Wasserstoff-Gehalt ausreichend zu erhöhen, um das Rohgas in das genannte Synthesegas umzuwandeln.

7. Verfahren nach Anspruch 4, dadurch gekennzeichnet, daß der Wasserstoff und das Kohlenmonoxid des Rohgases in Gegenwart eines für Schwefel unempfindlichen Katalysators in einer vorherigen direkten Methanbildungs-Reaktion ohne nennenswerten Zusatz von Wasser zur Reaktion gebracht wird, um eine teilweise Methanbildung des Rohrgases zu bewirken, und daß das resultierende Gas der genannten Vorbehandlungsstufe zugeführt wird.

8. Verfahren nach einem der Ansprüche 1 bis 4, dadurch gekennzeichnet, daß das genannte Zwischenproduktgas restlichen Wasserstoff und Kohlenmonoxid im Verhältnis von weniger als 3 sowie Wasser enthält, und daß das genannte Zwischenproduktgas nachbehandelt wird, indem das Wasser und das Kohlenmonoxid des Produktgases in Gegenwart eines für Schwefel unempfindlichen Katalysators in einer Wasser-Gas-Verschiebungs-Reaktion zur Reaktion gebracht wird, um das Wasserstoff-Kohlenmonoxid-Verhältnis darin auf wenigstens 3 anzuheben, bevor das Produktgas der Stufe zum Herauswaschen der saueren Gase zugeführt wird.

9. Verfahren nach Anspruch 1, dadurch gekennzeichnet, daß anstelle der zuletzt erwähnten Reaktion das Zwischenproduktgas einer kryogenen Methanabtrennung unterworfen wird, durch die abgetrenntes, zur direkten Methanbildungsstufe zurückgeführtes Kohlenmonoxid und Wasserstoff, getrennte sauer Gase und abgetrenntes methanreiches endgültiges Produktgas mit Rohrleitungsqualität erzeugt wird.

10. Verfahren nach Anspruch 9, dadurch gekennzeichnet, daß das abgetrennte endgültige Produktgas weitgehend verflüssigt wird.

**Revendications**

1. Un procédé pour transformer un gaz de synthèse contenant de l'hydrogène, de l'oxyde de carbon et un gaz contenant du soufre en un produit final qui est un ga riche in méthane, de qualité pour pipeline, dans lequel on fait réagir l'hydrogène et l'oxyde de carbone du gaz de synthèse en présence d'un premier catalyseur et en présence du gaz contenant du soufre dans une réaction de méthanation directe pour donner comme produit intermédiaire un gaz riche in méthane et contenant de l'hydrogène et de l'oxyde de carbone résiduels ainsi que des gaz acides;

on élimine ces gaz acides par lavage du gaz obtenu comme produit intermédiaire; et

on fait réagir l'hydrogène et l'oxyde de carbone résiduels présents dans le gaz lavé en présence d'un second catalyseur afin d'augmenter la teneur en méthane lavé et de réduire sa teneur en oxyde de carbone à pas plus d'environ 0,1% en volume et de le transformer ainsi en un produit final qui est un gaz riche en méthane, de qualité pour pipeline;

caractérisé en ce que le rapport de l'hydrogène à l'oxyde de carbone dans le gaz de synthèse est compris entre 0,9 et 1,3 et que le premier catalyseur est un catalyseur oxysulfure à base des éléments de transition tungstène, molybdène et vanadium.

2. Un procédé selon la revendication 1, caractérisé en ce que le premier catalyseur est un catalyseur sulfuré à base d'un ou plusieurs des éléments du groupe constitué par le tungstène, le molybdène et le vanadium, et supporté et/ou activé par un ou plusieurs éléments choisis dans le groupe Al, Si, B, Ce, Ti, Zr.

3. Un procédé selon la revendication 1 ou 2, caractérisé en ce qu'un matière première est gazéifiée de manière à produire ledit gaz de synthèse, contenant de l'hydrogène et de l'oxyde de carbone dans le rapport spécifié.

4. Un procédé selon la revendication 1 ou 2, caractérisé en ce qu'une matière première est gazéifiée de manière à produire un gaz brut contenant de l'hydrogène et de l'oxyde de carbone dans un rapport à

l'extérieur de l'intervalle de 0,9 à 1,3, et que le gaz brut est préconditionné de manière à ajuster le rapport de l'hydrogène à l'oxyde de carbone afin de produire ledit gaz de synthèse.

5. Un procédé selon la revendication 4, caractérisé en ce qu'une matière première est gazéifiée de manière à produire un gaz brut contenant de l'hydrogène et de l'oxyde de carbone dans un rapport supérieur à 1,3, ainsi que de l'anhydride carbonique, et que le gaz brut est préconditionné par réaction de l'hydrogène et de l'anhydride carbonique du gaz brut en présence d'un catalyseur insensible au soufre, dans une réaction inverse de la conversion du gaz par l'eau, afin de réduire la teneur en hydrogène et d'augmenter la teneur en oxyde de carbone dans une mesure suffisante pour transformer le gaz brut en ledit gaz de synthèse.

6. Un procédé selon la revendication 4, caractérisé en ce que le gaz brut contient de l'hydrogène et de l'oxyde de carbone dans un rapport inférieur à 0,9, ainsi que de l'eau, et que l'étape de préconditionnement comprend la réaction de l'oxyde de carbone et de l'eau du gaz brut en présence d'un catalyseur insensible au soufre, dans une réaction de conversion du gaz par l'eau, afin de réduire la teneur en oxyde de carbone et d'augmenter la teneur en hydrogène dans une mesure suffisante pour transformer le gaz brut en ledit gas de synthèse.

7. Un procédé selon la revendication 4, caractérisé en ce qu'on fait réagir l'hydrogène et l'oxyde de carbone du gaz brut en présence du catalysuer insensible au soufre dans une réaction préliminaire de méthanation directe sans addition importante d'eau pour effectuer une méthanation partielle du gaz brut et le gaz résultant est passé à ladite étape de préconditionnement.

8. Un procédé selon l'une quelconque des revendications 1 à 4, caractérisé en ce que ledit gaz obtenu comme produit intermédiaire contient de l'hydrogène et de l'oxyde de carbone résiduels dans un rapport de moins de 3, ainsi que de l'eau, et que l'on soumet ce gaz obtenu comme produit intermédiaire à un post-conditionnement en faisant réagir l'eau et l'oxyde de carbone de ce gaz en présence d'un catalyseur insensible au soufre, dans une réaction de conversion du gaz par l'eau, de manière à élever le rapport de l'hydrogène à l'oxyde de carbone dans ce gaz à une valeur d'au moins 3 avant l'introduction du gaz dans l'étape d'élimination des gaz acides par lavage.

9. Un procédé selon la revendication 1, caractérisé en ce qu'au lieu de la réaction mentionnée en dernier lieu, le gaz obtenue comme produit intermédiaire est soumis à une séparation cryogénique du méthane produisant de l'oxyde de carbone et de l'hydrogène séparés recyclés à l'étape de méthanation directe, des gaz acides séparés et un produit final séparé qui est un gaz riche en méthane, de qualité pour pipeline.

10. Un procédé selon la revendication 9, caractérisé ce que le gaz obtenu comme produit final séparé est substantiellement liquéfie.

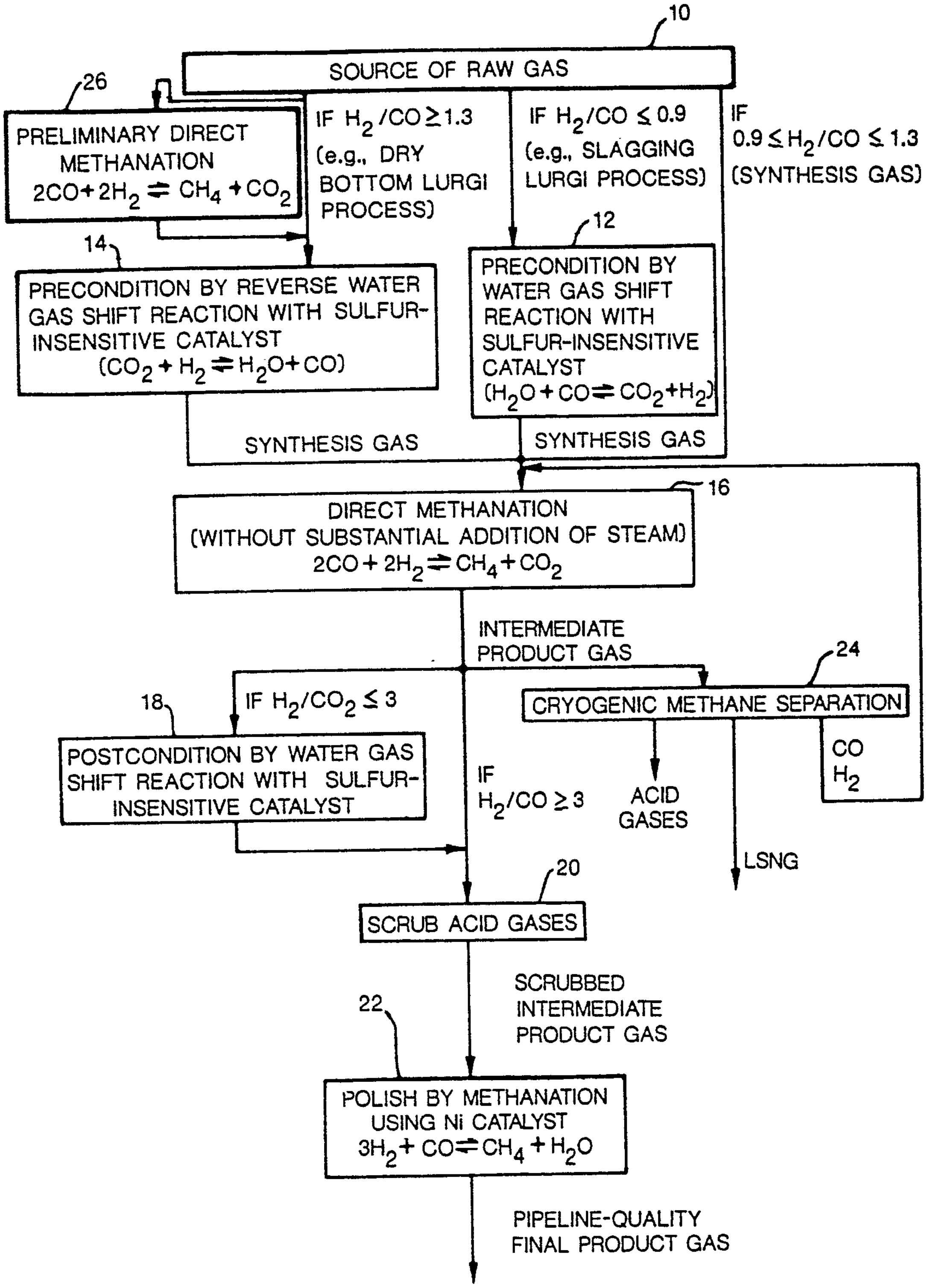
10
SOURCE OF RAW GAS
26
PRELIMINARY DIRECT METHANATION
2CO+2H2 ⇌ CH4+CO2
IF H2/CO ≥ 1.3
(e.g., DRY BOTTOM LURGI PROCESS)
IF H2/CO ≤ 0.9
(e.g., SLAGGING LURGI PROCESS)
IF 0.9 ≤ H2/CO ≤ 1.3
(SYNTHESIS GAS)
14
PRECONDITION BY REVERSE WATER GAS SHIFT REACTION WITH SULFUR-INSENSITIVE CATALYST
(CO2 + H2 ⇌ H2O + CO)
12
PRECONDITION BY WATER GAS SHIFT REACTION WITH SULFUR-INSENSITIVE CATALYST
(H2O + CO ⇌ CO2 + H2)
SYNTHESIS GAS
SYNTHESIS GAS
16
DIRECT METHANATION
(WITHOUT SUBSTANTIAL ADDITION OF STEAM)
2CO + 2H2 ⇌ CH4 + CO2
INTERMEDIATE PRODUCT GAS
24
CRYOGENIC METHANE SEPARATION
CO
H2
ACID GASES
LSNG
18
IF H2/CO2 ≤ 3
POSTCONDITION BY WATER GAS SHIFT REACTION WITH SULFUR-INSENSITIVE CATALYST
IF H2/CO ≥ 3
20
SCRUB ACID GASES
SCRUBBED INTERMEDIATE PRODUCT GAS
22
POLISH BY METHANATION USING Ni CATALYST
3H2 + CO ⇌ CH4 + H2O
PIPELINE-QUALITY FINAL PRODUCT GAS